(19) Europäisches Patentamt
European Patent Office
Office européen des brevets

(11) **EP 3 466 364 B1**

(12) **EUROPEAN PATENT SPECIFICATION**

(45) Date of publication and mention
of the grant of the patent:
**09.06.2021 Bulletin 2021/23**

(51) Int Cl.:
***A61B 18/18*** (2006.01)   ***A61N 5/02*** (2006.01)

(21) Application number: **16902938.6**

(22) Date of filing: **21.10.2016**

(86) International application number:
**PCT/CN2016/102870**

(87) International publication number:
**WO 2017/201954 (30.11.2017 Gazette 2017/48)**

(54) **ANTENNA ASSEMBLY FOR MICROWAVE ABLATION AND MICROWAVE ABLATION NEEDLE USING SAME**

ANTENNENANORDNUNG ZUR MIKROWELLENABLATION UND MIKROWELLENABLATIONSNADEL MIT VERWENDUNG DAVON

ENSEMBLE ANTENNE POUR ABLATION PAR MICRO-ONDES ET AIGUILLE D'ABLATION PAR MICRO-ONDES ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2016 CN 201610348040**

(43) Date of publication of application:
**10.04.2019 Bulletin 2019/15**

(73) Proprietor: **Surgnova Healthcare Technologies (Zhejiang) Co., Ltd.**
**Cixi, Zhejiang 315300 (CN)**

(72) Inventors:
• **HUANG, Wenxing**
**Beijing 100012 (CN)**
• **ZHAN, Dezhi**
**Beijing 100012 (CN)**
• **YAN, Yong**
**Beijing 100012 (CN)**
• **LI, Hui**
**Beijing 100012 (CN)**

(74) Representative: **Algemeen Octrooi- en Merkenbureau B.V.**
**P.O. Box 645**
**5600 AP Eindhoven (NL)**

(56) References cited:
**WO-A1-2016/029022   CN-A- 1 489 807**
**CN-A- 101 926 046   CN-A- 102 727 306**
**CN-A- 103 717 166   CN-A- 105 816 240**
**US-A- 4 700 716   US-A- 5 026 959**
**US-A1- 2007 049 917   US-A1- 2010 097 284**
**US-A1- 2011 077 635   US-A1- 2012 172 863**
**US-A1- 2014 155 881**

**Description**

Technical Field

This disclosure relates to the technical field of microwave treatment devices, in particular, to an antenna assembly for microwave ablation and a microwave ablation needle using the same.

Background Art

In recent years, microwave ablation gradually becomes an important treatment means for treating liver cancer. Microwave ablation uses the heat effect of microwaves in polar molecules, such as water, to allow the pathology area achieving a very high temperature instantaneously, so that the issue is solidified and necrosed by dewatering, thereby achieving a treatment purpose.

Concerning the design of microwave antenna, there are two types of ablation needles that are popular on the market currently. One type is ablation antennas without using choke technology, for example, that disclosed in Chinese Patent Application Publication with the Publication No. CN103142307A. The other type is ablation antennas using a choke ring (groove), for example, that disclosed in Chinese Patent Application Publication with the Publication No. CN104688335A, of which the main structure is shown in Fig. 1.

In the case that the choke technology is not used, some microwave will "escapes" backwards along the outside surface of the exterior conductor of the coaxial cable, thereby resulting in that the ablation zone becomes an ellipsoidal shape. The ablation antenna using the technology of a choke ring (groove) may inhibit the backward escaping of the microwave and obtain a rounder ablation zone. However, in order to stabilize the performance of the choke ring (groove), it is required to fill a high dielectric constant medium stable with respect to temperature, which results in that the circulation water cannot arrive at the head of the ablation needle. An excessively high temperature of the head of the needle tends to burn the ablation needle out, or it is even possible to cause crack of the ablation needle and cause medical accident.

US 2010/097284A1 discloses a microwave antenna assembly having a choke. US 2007/049917A1 discloses an antenna having a sleeve for microwave tumor ablation. US 4700716 discloses a microwave collinear antenna array applicator having metallic and dielectric cylinders for in situ or in vivo treatment of tumors. US 2014/155881 A1 discloses a microwave antenna having a choke. US 5026929A discloses a microwave radiator having ring conductors via a dielectric for warming therapy.

Summary

In view of above, an object of this disclosure is to provide an antenna assembly for microwave ablation and a microwave ablation needle using the same.

In order to achieve the above-mentioned object, in one aspect of this disclosure, this disclosure provides an antenna assembly for microwave ablation, comprising:

a radiator for emitting a microwave for ablation;
a coaxial cable for transmitting the microwave for ablation generated by a microwave generator to the radiator;
wherein an annular composite structure for inhibiting an electromagnetic wave propagated backwards along the coaxial cable is provided around the coaxial cable, wherein the annular composite structure comprises an annular nonmetallic layer and an annular metallic layer located outside the annular nonmetallic layer, wherein the annular metallic layer is electrically insulated from the coaxial cable,
wherein the antenna assembly further comprises a cooling channel for cooling the radiator,
wherein the annular metallic layer is formed on an exterior wall of the cooling channel provided around the coaxial cable by wrapping or adhering a metallic foil processed as a thin layer around the exterior wall of the cooling channel provided around the coaxial cable or by a sputtering process, an electroplating process or an electroless plating process, so that the annular metallic layer and the cooling channel of nonmetallic material together build the annular composite structure or a part of the annular composite structure.

As a preferred embodiment, where a direction where the radiator lies is a front end of the metallic layer and an opposite direction thereof is a back end of the metallic layer, a length $L_1$ of the metallic layer in the annular composite structure and a distance $L_2$ between a feeding point and the back end of the metallic layer satisfy the following relationship:

$$\sqrt{\epsilon_1}L_1 - \sqrt{\epsilon_2}[L_2 - (L_1 - L_2)] \approx {}^C\!/_{2f} \quad (1)$$

wherein $\varepsilon_1$ is a relative permittivity of a human tissue, $\varepsilon_2$ is a relative permittivity of a material of the annular nonmetallic layer, C is the light velocity in vacuum, and f is a frequency of the microwave; wherein the difference between the both sides of the formula mentioned above is within $\pm 20\%$.

As a preferred embodiment, a distance $L_3$ from the front end of the metallic layer to the most front end of the radiator and the $L_1$ further satisfy the following relationship:

$$L_1 \approx L_3 \ (2)$$

wherein the difference between the both sides of the formula mentioned above is within $\pm 20\%$.

As a preferred embodiment, material of the annular metallic layer is copper, iron, aluminum, gold, silver, palladium, platinum, tin, nickel, zinc, or an alloy thereof.

As a preferred embodiment, the annular composite structure has a total thickness ranging from 0.001 to 2mm.

As a preferred embodiment, for an antenna assembly having a frequency of 2.45 GHz, the length L1 of the annular metallic layer is between 5 and 25mm.

As a preferred embodiment, the cooling channel is preferably able to transport a cooling medium to the most front end of the radiator so as to cool the whole radiator.

As a preferred embodiment, the cooling channel is of nonmetallic material, preferably made of PTFE material.

As a preferred embodiment, a radiation zone of the antenna assembly is not filled by any high dielectric constant solid medium having a relative permittivity of 25 or more.

As a preferred embodiment, the radiator is a metallic "cap" or an extended section of an inner core of the coaxial cable.

As a preferred embodiment, the antenna assembly further comprises a temperature detector for detecting a temperature of the radiator.

In another aspect of this disclosure, this disclosure further provides a microwave ablation needle, which comprises the antenna assembly as mentioned above.

As can be seen from the above-mentioned technical solutions, the antenna assembly and the microwave ablation needle of this disclosure has the following advantageous effects: (1) that the annular composite structure may inhibits a microwave propagated backwards along the coaxial cable and an exterior wall of a water inlet pipe effectively; and (2) that it is unnecessary to fill a stabilizing medium in a radiation zone by a choke technology using the annular composite structure, so that the circulation water enters the radiation zone through the annular composite structure (choke structure), and the temperatures of the head of the ablation needle and the needle stem may be controlled effectively, so as to avoid burnout of the ablation needle and medical accident due to an excessively high temperature.

Brief Description of Drawings

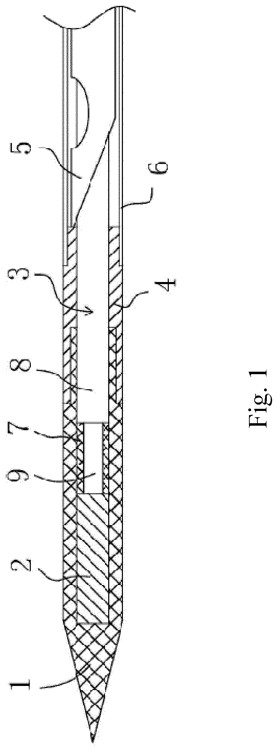

Fig. 1 is a schematic drawing of a main structure of an antenna assembly for microwave ablation in the related art;
Fig. 2 is a schematic drawing of a main structure of an antenna assembly for microwave ablation of this disclosure;
Fig. 3 and Fig. 4 are distribution diagrams of the absorption field of the ablation antenna in the case where there is no annular composite structure and in the case where there is an annular composite structure, respectively.

Detailed Embodiments

In order to making the purposes, technical solutions and advantages of this disclosure clearer, this disclosure is further described in details by using specific examples and referring to drawings.

This disclosure discloses an antenna assembly for microwave ablation, comprising: a radiator and a coaxial cable, wherein the radiator is for emitting a microwave for ablation and the coaxial cable is for transmitting the microwave for ablation generated by a microwave generator to the radiator; wherein an annular composite structure for inhibiting an electromagnetic wave propagated backwards along the coaxial cable is provided around the coaxial cable, wherein the annular composite structure comprises an annular nonmetallic layer and an annular metallic layer located outside the annular nonmetallic layer, wherein the annular metallic layer is electrically insulated from the coaxial cable. As a preferred embodiment, the annular composite structure may comprise the two layers mentioned above only, or may further comprise more layers, such as a further nonmetallic layer outside the annular metallic layer.

The annular metallic layer may be made by using various conductive metals, for example, copper, iron, aluminum, gold, silver, palladium, platinum, tin, nickel, zinc, or an alloy thereof, in which copper, for example, a copper foil, a material plated by copper by sputtering, a copper-plated material, is used preferably, for example, by wrapping or adhering a metallic foil processed as a thin layer around the exterior wall of the nonmetallic layer, or by forming a thin metallic layer on the outside surface of the nonmetallic layer by a process, such as sputtering, or by forming a thin metallic layer on the outside surface of the nonmetallic layer by a process, such as electroplating or electroless plating. The shape of the annular metallic layer changes with the outline of the water inlet pipe. Generally, it has an annular shape, with a thickness ranging from 0.001 to 2mm, preferably 0.05mm, and an length which is not limited but is preferably about half (in human tissue) of the wavelength of the electromagnetic wave propagated backwards along the coaxial cable. For a frequency of 2.45 GHz, the length ranges from 5 to 25mm, preferably 11mm.

The annular composite structure may be provided as a ring around the coaxial cable, which may be provided against the coaxial cable closely, or may also be provided to keep a distance to the coaxial cable, for example, a distance being twice of the diameter of the coaxial cable. Anyway, it must be ensured that the annular metallic layer in the annular composite structure is electrically insulated from the coaxial cable. The total thickness of the annular composite structure is preferably ranging from 0.001 to 2mm. As a preferred embodiment, a cooling channel may be formed outside the coaxial cable by using a form of an encircled nonmetallic flexible tube. The cooling channel is used to pass the cooling medium flowing in through the interior wall gap and pass the cooling medium flowing out through the exterior wall gap. The cooling medium is used to cool a part or all of the radiation zone including the radiator. In this case, it is possible to use the cooling channel as the nonmetallic layer of the annular composite structure, and to form the annular composite structure together by a metallic layer provided on the outside surface thereof. In a preferred example, the cooling channel is of PTFE (polytetrafluoroethylene) material. In order to enhance the strength thereof, a stainless steel pipe may be used in a connection section thereof. However, the annular metallic layer must be formed on the outside surface of the cooling channel of the PTFE material.

As another novel point of this disclosure, the radiation zone of the antenna assembly of this disclosure may be not filled by any high dielectric constant medium, for example, high permittivity ceramic materials having a relative permittivity of 25 or more, such as zirconium oxide, or the like.

In the above-mentioned antenna assembly, the radiator may be a metallic "cap", for example, a copper "cap", or may also just be an extended section of an inner core of the coaxial cable. In actual use, lengths of the both are designed to be the same.

When a direction where the radiator lies, i.e. the direction of the head of the antenna assembly, is a front end of the metallic layer and an opposite direction thereof is a back end of the metallic layer, a length $L_1$ of the annular metallic layer and a distance $L_2$ between a feeding point and the back end of the annular metallic layer substantially satisfy the following relationship:

$$\sqrt{\epsilon_1}L_1 - \sqrt{\epsilon_2}[L_2 - (L_1 - L_2)] \approx {}^C\!/_{2f} \ (1)$$

wherein $\epsilon_1$ is a relative permittivity of a human tissue, $\epsilon_2$ is a relative permittivity of a material of the annular nonmetallic layer, C is the light velocity in vacuum, and f is a frequency of the microwave. The difference between the both sides of the formula mentioned above is within $\pm 40\%$, preferably within $\pm 20\%$.

Further, the length $L_1$ of the annular metallic layer and a distance $L_3$ from the front end of the annular metallic layer to the front end of the radiator substantially satisfy the following relationship:

$$L_1 \approx L_3 \ (2)$$

wherein the difference between the both sides of the formula mentioned above is within $\pm 40\%$, preferably within $\pm 20\%$.

The design of the above-mentioned antenna allows the cooling medium, for example, the circulation water arriving at the head of the antenna (copper cap). Therefore, after the ablation has been carried out for a period of time, the human tissue around the antenna is carbonized, and the relative permittivity thereof decreases. Then, instead of the human tissue, the circulation water makes $\epsilon_1$ to be kept at a relatively high value, thereby keeping the formula (1) still establishing, and maintaining the choke property of the antenna. Thereby, by using the annular composite structure for choking, the antenna assembly corresponds to a symmetrical full wave dipole antenna, which has a more uniform near field distribution than a half wave dipole antenna.

In a preferred example, the antenna assembly further comprises a temperature detector for detecting a temperature of the radiator. In this case, the temperature detector is preferably connected to a control circuit. When a temperature exceeding a certain threshold is detected, means such as increasing the flow rate of the cooling medium in the cooling channel, or decreasing the emission power of the radiator, are used to prevent excessive rise of the radiator temperature and thus burnout of the radiator, and to prevent damage to the human body.

In another aspect of this disclosure, this disclosure further provides a microwave ablation needle, which comprises the antenna assembly as mentioned above.

The technical solutions of this disclosure are further described below by referring to drawings.

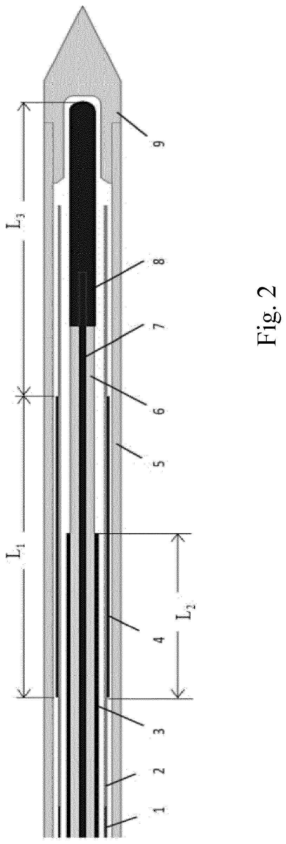

Fig. 2 is a schematic drawing of a structure of an antenna assembly of a preferred example of this disclosure. In the drawing, the components successively are a stainless steel water inlet pipe section 1, a PTFE water inlet pipe section 2, a coaxial cable exterior conductor 3, a copper foil 4, a glass fiber outside pipe 5, a coaxial cable medium layer 6, a coaxial cable inner core 7, a copper "cap" 8 and a ceramic prick head 9. Among these, the water inlet pipe comprises the stainless steel water inlet pipe section 1 and the PTFE water inlet pipe section 2, wherein the stainless steel water inlet pipe section 1 is connected to the PTFE water inlet pipe section 2 in a radiation zone of the antenna. The circulation water flows to the antenna head through the gap between the water inlet pipe and the coaxial cable, and flows out from the gap between the glass fiber outside pipe 5 and the water inlet pipe. The copper foil 4 is against the exterior wall of the PTFE water inlet pipe section 2 closely, and the copper "cap" 8 is welded to the coaxial cable inner core 7.

If there is no copper foil 4 in the water inlet pipe in the antenna structure, after the microwave arrives at the feeding point (the end of the coaxial cable exterior conductor), a part of the microwave is transmitted forwards along the coaxial cable inner core 7 and radiates into the ambient tissue or another medium by the copper "cap" 8, while the other part is transmitted backwards along the outside surface of the coaxial cable exterior conductor 3 in the outside tissue, circulation water, PTFE of the inner pipe and glass fiber of the outside pipe and radiates into the ambient tissue at the same time. This will result in that the microwave ablation zone becomes an ellipsoidal shape. In the antenna assembly of this disclosure, since a length of copper foil covers the water inlet pipe, the microwave propagated backwards would be divided into two components. One microwave component is transmitted in the tissue along the outside wall of the copper foil 4, while the other microwave component is propagated along the inner wall of the copper foil 4 and the coaxial cable exterior conductor 3 in the PTFE water inlet pipe section 2. The energy of the microwave propagated in the circulation water is relatively few and is negligible.

Since the relative dielectric constant of the human tissue (about 40) is many times higher than that of the PTFE medium (about 2.5), the phase difference between the two microwave components is larger when the propagation distance along the copper foil 4 is larger. When a copper foil 4 with an appropriate length (slightly larger than a half of the wave length of the microwave in the human tissue) is used, it is possible to allow the two microwave components to have a half-cycle difference and to be opposite with each other. At the same time, the position of the copper foil with respect to the feeding point is adjusted properly, so that the amplitudes of the two microwave components are the same. Thus, when the two microwave components meet at the end of the copper foil, they would be cancelled out with each other due to the same amplitudes and the opposite phases. The microwave will be cut off at the end of the copper foil 4 and will not continue to be propagated backwards along the coaxial cable.

Thus, the microwave will be concentrated in the zone around the copper foil and the copper "cap" to perform ablation. Therefore, a rounder ablation zone may be obtained by this kind of novel ablation antenna.

In the technical solutions mentioned above, a person skilled in the art also can make simple modification or replacement to them. Examples are as follows.

(1) The main function of the copper "cap" is to extend and reinforce the coaxial cable inner core, and it is possible to just use a coaxial cable inner core.
(2) Another nonmetallic material having a low dielectric constant (less than 3) may be used for the PTEF water inlet pipe section 2, while the length and position of the copper foil need to be adjusted slightly.
(3) The materials and thicknesses of the outside pipe and the prick head may be selected according to practical conditions. However, it should be ensured that the water inlet pipe and the outside pipe are of nonmetallic material in the radiation zone of the antenna.
(4) Another process may be used to cover the water inlet pipe with the copper foil, for example, by plating a film of metal on the water inlet pipe.

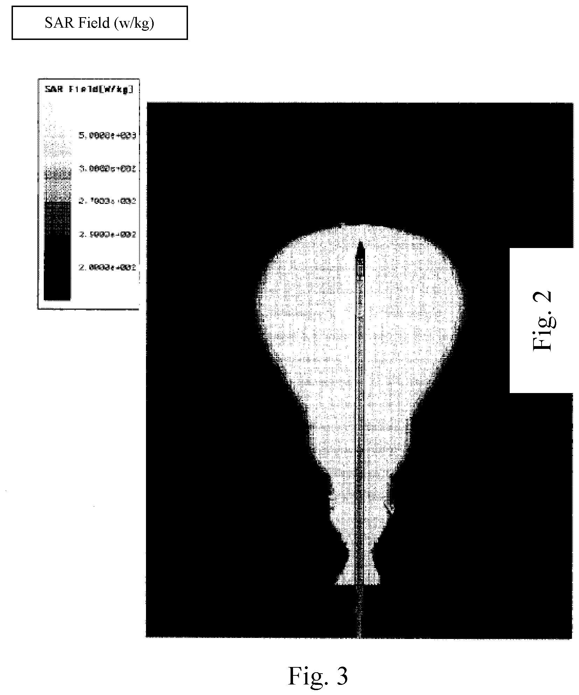

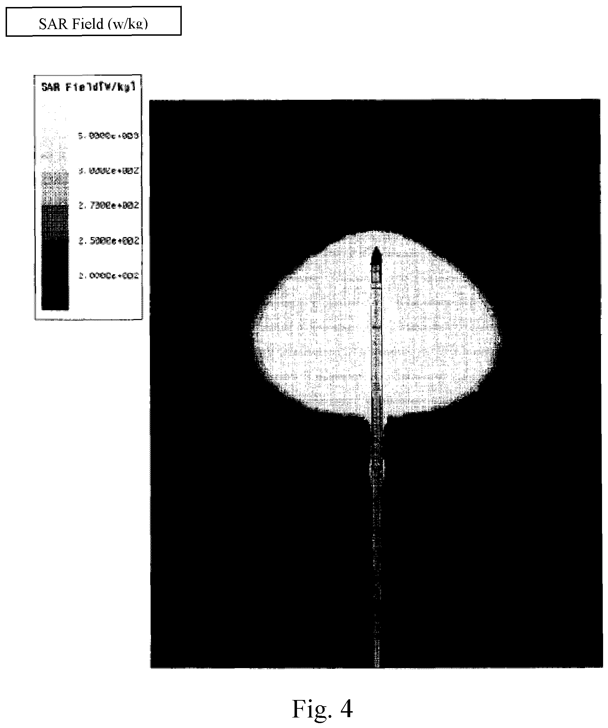

By experimental validation, the distributions of the absorption fields in both the case where there is no annular composite structure and the case where there is the annular composite structure are as shown in Fig. 3 and Fig. 4. As can be seen therefrom, the antenna design of this disclosure may inhibit effectively the backward propagation of the microwave along the coaxial cable exterior wall and obtain a relatively ideal spherical ablation zone. Meanwhile, in the ablation antenna of this disclosure, it is not required to fill any stabilizing medium in the radiation zone. The circulation water is allowed to enter the radiation zone, so that the temperature of the head the ablation needle may be controlled effectively, so as to avoid burnout of the ablation needle and medical accident due to an excessively high temperature.

The purposes, technical solutions and advantageous effects of this disclosure are further described in details by the above-mentioned specific examples. It should be understood that the contents above are merely specific examples of this disclosure ant would not be used to limit this disclosure.

**Claims**

1.  An antenna assembly for microwave ablation, comprising:

    a radiator for emitting a microwave for ablation;
    a coaxial cable for transmitting the microwave for ablation generated by a microwave generator to the radiator;
    wherein an annular composite structure for inhibiting an electromagnetic wave propagated backwards along the coaxial cable is provided around the coaxial cable, wherein the annular composite structure comprises an annular nonmetallic layer and an annular metallic layer located outside the annular nonmetallic layer, wherein the annular metallic layer is electrically insulated from the coaxial cable,
    wherein the antenna assembly further comprises a cooling channel provided around the coaxial cable for cooling the radiator, wherein the cooling channel is of nonmetallic material, and
    wherein the annular metallic layer is formed on an exterior wall of the cooling channel, so that the annular metallic layer and the cooling channel of nonmetallic material together build the annular composite structure or a part of the annular composite structure.

2.  The antenna assembly according to claim 1, **characterized in that** where a direction where the radiator lies is a front end of the annular metallic layer and an opposite direction thereof is a back end of the annular metallic layer, a length $L_1$ of the annular metallic layer in the annular composite structure and a distance $L_2$ between a feeding point and the back end of the annular metallic layer satisfy the following relationship:

$$\sqrt{\epsilon_1}L_1 - \sqrt{\epsilon_2}[L_2 - (L_1 - L_2)] \approx \frac{C}{2f}$$

    wherein $\epsilon_1$ is a relative permittivity of a human tissue, $\epsilon_2$ is a relative permittivity of a material of the annular nonmetallic layer, C is the light velocity in vacuum, and f is a frequency of the microwave; wherein the difference between the both sides of the formula mentioned above is within ±40%.

3.  The antenna assembly according to claim 2, **characterized in that** the difference between the both sides of the formula mentioned above is within ±20%.

4.  The antenna assembly according to claim 2, **characterized in that** a distance $L_3$ from the front end of the metallic layer to the most front end of the radiator and the $L_1$ further satisfy the following relationship:

$$L_1 \approx L_3$$

    wherein the difference between the both sides of the formula mentioned above is within ±40%,
    preferably, the difference between the both sides of the formula mentioned above is within ±20%.

5.  The antenna assembly according to claim 2, **characterized in that** for an antenna assembly having a frequency of 2.45 GHz, the length L1 of the annular metallic layer is between 5 mm and 25mm.

6.  The antenna assembly according to claim 1, **characterized in that** a material of the annular metallic layer is copper, iron, aluminum, gold, silver, palladium, platinum, tin, nickel, zinc, or an alloy thereof.

7.  The antenna assembly according to claim 1, **characterized in that** the annular composite structure has a total thickness ranging from 0.001 to 2mm.

8.  The antenna assembly according to claim 1, **characterized in that** the cooling channel is able to transport a cooling medium to the most front end of the radiator so as to cool the whole radiator.

9.  The antenna assembly according to claim 1, **characterized in that** the cooling channel is made of PTFE material.

10. The antenna assembly according to claim 1, **characterized in that** a radiation zone of the antenna assembly is not filled by any high dielectric constant solid medium having a relative permittivity of 25 or more.

11. The antenna assembly according to claim 1, **characterized in that** the radiator is formed by a metallic cap or by an extended section of an inner core of the coaxial cable.

**12.** The antenna assembly according to claim 1, **characterized in that** the antenna assembly further comprises a temperature detector for detecting a temperature of the radiator.

**13.** The antenna assembly according to claim 1, wherein the annular metallic layer is formed on an exterior wall of the cooling channel a) by wrapping or adhering a metallic foil processed as a thin layer around the exterior wall of the cooling channel or b) by a sputtering process, an electroplating process or an electroless plating process.

**14.** A microwave ablation needle, comprising the antenna assembly according to any of claims 1 to 13.


**Patentansprüche**

**1.** Antennenanordnung zur Mikrowellenablation, umfassend:

einen Strahler zum Emittieren von Mikrowellen zur Ablation;
ein Koaxialkabel zum Übertragen der Mikrowellen zur Ablation, die durch einen Mikrowellengenerator generiert wurden, an den Strahler;
wobei eine kranzförmige Verbundstruktur, um eine elektromagnetische Welle daran zu hindern, sich entlang des Koaxialkabels rückwärts auszubreiten, um das Koaxialkabel herum bereitgestellt wird, wobei die kranzförmige Verbundstruktur eine kranzförmige nichtmetallische Schicht und eine kranzförmige metallische Schicht umfasst, die sich außerhalb der kranzförmigen nichtmetallischen Schicht befindet, wobei die kranzförmige metallische Schicht von dem Koaxialkabel elektrisch isoliert ist,
wobei die Antennenanordnung des Weiteren einen Kühlkanal umfasst, der um das Koaxialkabel herum bereitgestellt wird, um den Strahler zu kühlen, wobei der Kühlkanal aus nichtmetallischem Material ist, und
wobei die kranzförmige metallische Schicht auf einer Außenwand des Kühlkanals gebildet ist, so dass die kranzförmige metallische Schicht und der Kühlkanal des nichtmetallischen Materials zusammen die kranzförmige Verbundstruktur oder einen Teil der kranzförmigen Verbundstruktur bilden.

**2.** Antennenanordnung nach Anspruch 1, **dadurch gekennzeichnet, dass**, wenn eine Richtung, in welcher der Strahler liegt, ein vorderes Ende der kranzförmigen metallischen Schicht ist, und eine Gegenrichtung davon ein hinteres Ende der kranzförmigen metallischen Schicht ist, eine Länge $L_1$ der kranzförmigen metallischen Schicht in der kranzförmigen Verbundstruktur und ein Abstand $L_2$ zwischen einem Speisepunkt und dem hinteren Ende der kranzförmigen metallischen Schicht die folgende Beziehung erfüllt:

$$\sqrt{\epsilon_1}L_1 - \sqrt{\epsilon_2}[L_2 - (L_1 - L_2)] \approx C/_{2f}$$

wobei $\epsilon_1$ die relative Permittivität eines menschlichen Gewebes ist, $\epsilon_2$ die relative Permittivität eines Materials der kranzförmigen nichtmetallischen Schicht ist, C die Lichtgeschwindigkeit im Vakuum ist, und f eine Frequenz der Mikrowelle ist; wobei die Differenz zwischen beiden Seiten der oben genannten Formel innerhalb $\pm$ 40 % liegt.

**3.** Antennenanordnung nach Anspruch 2, **dadurch gekennzeichnet, dass** die Differenz zwischen den beiden Seiten der oben genannten Formel innerhalb von $\pm$ 20 % liegt

**4.** Antennenanordnung nach Anspruch 2, **dadurch gekennzeichnet, dass** ein Abstand $L_3$ von dem vorderen Ende der metallischen Schicht zu dem am weitesten vorne befindlichen Ende des Strahlers und dem $L_1$ des Weiteren die folgende Beziehung erfüllen:

$$L_1 \approx L_3$$

wobei die Differenz zwischen den beiden Seiten der oben genannten Formel innerhalb $\pm$ 40 % liegt,
wobei die Differenz zwischen den beiden Seiten der oben genannten Formel vorzugsweise innerhalb $\pm$ 20 % liegt.

**5.** Antennenanordnung nach Anspruch 2, **dadurch gekennzeichnet, dass** die Länge $L_1$ der kranzförmigen metallischen Schicht für eine Antennenanordnung mit einer Frequenz von 2,45 GHz zwischen 5 mm und 25 mm liegt.

**6.** Antennenanordnung nach Anspruch 1, **dadurch gekennzeichnet, dass** ein Material der kranzförmigen metallischen Schicht Kupfer, Eisen, Aluminium, Gold, Silber, Palladium, Platin, Zinn, Nickel, Zink oder eine Legierung davon ist.

**7.** Antennenanordnung nach Anspruch 1, **dadurch gekennzeichnet, dass** die kranzförmige Verbundstruktur eine Gesamtdicke hat, die im Bereich von 0,001 bis 2 mm liegt.

**8.** Antennenanordnung nach Anspruch 1, **dadurch gekennzeichnet, dass** der Kühlkanal in der Lage ist, ein Kühlmedium an das am weitesten vorne liegende Ende des Strahlers zu transportieren, um so den gesamten Strahler zu kühlen.

**9.** Antennenanordnung nach Anspruch 1, **dadurch gekennzeichnet, dass** der Kühlkanal aus PTFE-Material gefertigt ist.

**10.** Antennenanordnung nach Anspruch 1, **dadurch gekennzeichnet, dass** eine Strahlungszone der Antennenanordnung nicht durch irgendein festes Medium mit hoher Dielektrizitätskonstante mit einer relativen Permittivität von 25 oder mehr gefüllt ist.

**11.** Antennenanordnung nach Anspruch 1, **dadurch gekennzeichnet, dass** der Strahler durch eine metallische Kappe oder ein erweitertes Segment eines inneren Kerns des Koaxialkabels gebildet ist.

**12.** Antennenanordnung nach Anspruch 1, **dadurch gekennzeichnet, dass** die Antennenanordnung des Weiteren einen Temperaturdetektor zum Detektieren einer Temperatur des Strahlers umfasst.

**13.** Antennenanordnung nach Anspruch 1, wobei die kranzförmige metallische Schicht auf einer Außenwand des Kühlkanals gebildet wird durch a) Wickeln oder Kleben einer als Dünnschicht verarbeiteten Metallfolie um die Außenwand des Kühlkanals oder b) durch einen Sputterprozess, einen Elektroplattierprozess oder einen stromlosen Plattierprozess.

**14.** Mikrowellenablationsnadel, umfassend die Antennenanordnung nach einem der Ansprüche 1 bis 13.

**Revendications**

**1.** Ensemble antenne pour une ablation par micro-ondes, comprenant :

un radiateur pour émettre une micro-onde pour l'ablation ;
un câble coaxial pour transmettre la micro-onde pour l'ablation générée par un générateur de micro-ondes au radiateur ;
dans lequel une structure composite annulaire pour inhiber une onde électromagnétique propagée vers l'arrière le long du câble coaxial est prévue autour du câble coaxial, dans lequel la structure composite annulaire comprend une couche non métallique annulaire et une couche métallique annulaire située à l'extérieur de la couche non métallique annulaire, dans lequel la couche métallique annulaire est isolée électriquement du câble coaxial, dans lequel l'ensemble antenne comprend en outre un canal de refroidissement prévu autour du câble coaxial pour refroidir le radiateur, dans lequel le canal de refroidissement est en matériau non métallique, et
dans lequel la couche métallique annulaire est formée sur une paroi extérieure du canal de refroidissement, de sorte que la couche métallique annulaire et le canal de refroidissement en matériau non métallique construisent ensemble la structure composite annulaire ou une partie de la structure composite annulaire.

**2.** Ensemble antenne selon la revendication 1, **caractérisé en ce que**, lorsqu'une direction dans laquelle se trouve le radiateur est une extrémité avant de la couche métallique annulaire et une direction opposée de celle-ci est une extrémité arrière de la couche métallique annulaire, une longueur $L_1$ de la couche métallique annulaire dans la structure composite annulaire et une distance $L_2$ entre un point d'alimentation et l'extrémité arrière de la couche métallique annulaire satisfont la relation suivante :

$$\sqrt{\epsilon_1}L_1 - \sqrt{\epsilon_2}[L_2 - (L_1 - L_2)] \approx {}^C\!/_{2f}$$

où $\epsilon_1$ est une permittivité relative d'un tissu humain, $\epsilon_2$ est une permittivité relative d'un matériau de la couche non métallique annulaire, C est la vitesse de la lumière dans le vide, et f est une fréquence de la micro-onde ; où la différence entre les deux côtés de la formule mentionnée ci-dessus est de $\pm$ 40%.

3. Ensemble antenne selon la revendication 2, **caractérisé en ce que** la différence entre les deux côtés de la formule mentionnée ci-dessus est de $\pm$ 20%.

4. Ensemble antenne selon la revendication 2, **caractérisé en ce qu'**une distance $L_3$ de l'extrémité avant de la couche métallique à l'extrémité la plus en avant du radiateur et $L_1$ satisfont en outre la relation suivante :

$$L_1 \approx L_3$$

où la différence entre les deux côtés de la formule mentionnée ci-dessus est de $\pm$ 40%,
de préférence, la différence entre les deux côtés de la formule mentionnée ci-dessus est de $\pm$ 20%.

5. Ensemble antenne selon la revendication 2, **caractérisé en ce que**, pour un ensemble antenne ayant une fréquence de 2,45 GHz, la longueur L1 de la couche métallique annulaire est comprise entre 5 mm et 25 mm.

6. Ensemble antenne selon la revendication 1, **caractérisé en ce qu'**un matériau de la couche métallique annulaire est le cuivre, le fer, l'aluminium, l'or, l'argent, le palladium, le platine, l'étain, le nickel, le zinc ou un alliage de ceux-ci.

7. Ensemble antenne selon la revendication 1, **caractérisé en ce que** la structure composite annulaire a une épaisseur totale se trouvant dans la plage allant de 0,001 à 2 mm.

8. Ensemble antenne selon la revendication 1, **caractérisé en ce que** le canal de refroidissement est capable de transporter un milieu de refroidissement vers l'extrémité la plus en avant du radiateur de manière à refroidir tout le radiateur.

9. Ensemble antenne selon la revendication 1, **caractérisé en ce que** le canal de refroidissement est réalisé en matériau PTFE.

10. Ensemble antenne selon la revendication 1, **caractérisé en ce qu'**une zone de rayonnement de l'ensemble antenne n'est remplie par aucun milieu solide à constante diélectrique élevée ayant une permittivité relative supérieure ou égale à 25.

11. Ensemble antenne selon la revendication 1, **caractérisé en ce que** le radiateur est formé par un bouchon métallique ou par une section étendue d'un noyau interne du câble coaxial.

12. Ensemble antenne selon la revendication 1, **caractérisé en ce que** l'ensemble antenne comprend en outre un détecteur de température pour détecter une température du radiateur.

13. Ensemble antenne selon la revendication 1, dans lequel la couche métallique annulaire est formée sur une paroi extérieure du canal de refroidissement a) en enroulant ou en collant une feuille métallique traitée comme une couche mince autour de la paroi extérieure du canal de refroidissement ou b) par un procédé de pulvérisation, un procédé de galvanoplastie ou un procédé de dépôt autocatalytique.

14. Aiguille d'ablation par micro-ondes, comprenant l'ensemble antenne selon l'une des revendications 1 à 13.

Fig. 1

Fig. 2

SAR Field (w/kg)

Fig. 2

Fig. 3

SAR Field (w/kg)

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103142307 A
- CN 104688335 A
- US 2010097284 A1
- US 2007049917 A1
- US 4700716 A
- US 2014155881 A1
- US 5026929 A